# EUROPÄISCHE PATENTANMELDUNG

(11) **EP 2 057 994 A1**
(43) Veröffentlichungstag der Anmeldung: **13.05.2009**
(21) Anmeldenummer: 07120081.0
(22) Anmeldetag: 06.11.2007
(51) Int. Cl.: A61K 36/33, A61P 1/04, A61P 3/00

(54) **Feigenkakteen-Extraktzubereitung**

(71) Anmelder: Finzelberg GmbH & Co. KG, 56626 Andernach (DE)
(72) Erfinder: Pischel, Ivo, 53547 Rossbach (DE); Feistel, Björn, Dr., 56626 Andernach (DE); Walbroel, Bernd, 53639 Königswinter (DE)
(74) Vertreter: Schreiber, Christoph

(57) **Zusammenfassung**

Verfahren zur Herstellung einer Extraktzubereitung von Feigenkakteen, umfassend die Schritte:
- Extrahieren von Pflanzenteilen, ausgewählt aus Früchten, Fruchtteilen, Cladoden, Cladodenteilen und Mischungen davon mit einem Extraktionsmittel enthaltend Wasser und 0 bis 70 Gew-% Alkohole
- zumindest teilweises Entfernen des Extraktionsmittels
Verwendung dieser Extraktzubereitung zur Herstellung eines Lebensmittels, eines diätetischen Lebensmittels, eines Nahrungsergänzungs- oder Arzneimittels zur Prophylaxe oder Behandlung von Diabetes mellitus II, zur Steigerung der körpereigenen Insulinausschüttung, zur günstigen Beeinflussung von Parametern des metabolischen Syndroms, zur günstigen Beeinflussung des Blutglucosespiegels, zur günstigen Beeinflussung des Blutlipidprofils, sowie zur Behandlung von Störungen des Magensäurehaushaltes und säureabhängiger Ulcera in Ventriculus und Duodenum.

## Beschreibung

Die vorliegende Erfindung betrifft Feigenkakteen-Extraktzubereitungen, Verfahren zu ihrer Herstellung und ihre Verwendung. Die Feigenkakteen-Extraktzubereitungen haben einen positiven Einfluss auf den Blutglucosegehalt und können u.a. zur Behandlung und Prävention von Diabetes mellitus Typ 2 angewandt werden.

Der Feigenkaktus (Opuntia spp., Opuntia ficus indica) wird seit Jahrhunderten in der neuen Welt, in Süd-, Mittel- und Nordamerika, aber auch in den Mittelmeerländern, Nordafrika und anderen Gebieten der Erde in der Volksmedizin, zur Behandlung von Krankheiten, zur Beschwerdenlinderung, sowie zur Vorbeugung von Stoffwechselleiden verwendet.

Stintzing FC und Carle R (Cactus stems (Opuntia spp.) A review on their chemistry, technology, and uses. Mol. Nutr. Food Res. 2005, 49, 175-194) beschreiben eine Reihe von Anwendungen der Kakteenstämme (Cladoden), wie antioxidative, entzündungshemmende (auch bei Ulzera), analgetische, hypoglykämische, antidiabetische, sowie Blutlipid und -cholesterin-senkende Effekte. Den Kaktusfeigen, den essbaren Früchten der Opuntien, werden durch ihren Gehalt an gefärbten Betalainderivaten bestimmte Anwendungen als Nahrungsmittel, insbesondere als Lebensmittelfarbstoffkonzentrate und therapeutische Heilmittel zugeschrieben. So zeigt das Review von Livrea MA und Tesoriere L (Health Benefit and Bioactive Components of the Fruits from Opuntia ficus-indica. J. PACD 2006, 73-90) vor allem in der Anwendung der essbaren Fruchtteile oder des -saftes antioxidative Aktivität, neben kardioprotektiven, anti-ulzera und hepatoprotektiven Effekten, sowie protektive Wirkung auf Nervengewebe und eine Krebsprävention. Als bioaktive Substanzen werden die antioxidativen Betalainderivate mit Radikalfängerfunktion angeführt. Den Früchten oder deren Teilen wurden bisher keine antidiabetischen oder hypoglykämischen Aktivitäten zugeschrieben.

Die traditionale Anwendung von Opuntien bei Diabetes Mellitus 2 beschränkt sich auf die Verwendung von frischen Cladoden (junge Kaktustriebe), die zumeist gekocht, gebraten oder gegrillt, in Mengen von bis zu 500 Gramm gegessen werden (Frati AC, Xilotl Diaz N, Altamirano P, Ariza R, Lopez-Ledesma R.; The effect of two sequential doses of Opuntia streptacantha upon glycemia. Arch Invest Med (Mex)., 1991, 333-6.)

Getrocknete und gepulverte Cladoden in Form pharmazeutischer Darreichungsformen, wie Tabletten und Kapsel, haben in relativ hohen Dosen nur eine Blutlipid-senkende Wirkung und werden ausschließlich hierfür vermarktet (www.neopuntia.com).

Nicht genauer definierte Extrakte der Cladoden, hergestellt mit organischen Lösungsmitteln, wurden in der Literatur mit einer hypoglykämische Wirkung beschrieben. (Trejo-Gonzalez A et al. (A purified extract from prickly pear cactus (Opuntia fuliginosa) controls experimentally induced diabetes in rats. Journal of Ethnopharmacology, 1996, 27-33.

In der Literatur, einschließlich der Patentliteratur, sind Pflanzenextrakte aus den Cladoden der Feigenkakteen außer den oben genannten für die Anwendungen bei Alkoholkater (KR 2004-0094173), als Neuroprotektivum (KR 2003-0035974), Immunstimulans (KR 2002-0092021), bei dermalen Problemen (JP 2002-0072130; KR 2003-0023398) und als Mikrobiozid (US 3,860,710; KR 2003-0023398) beschrieben. Die Früchte (Kaktusfeigen) werden zur Gewinnung der zur Lebensmittelfärbung verwendeten Farbstoffe (Betalaine u.a.) extraktiert und konzentriert (KR 2003-0035974; Moßhammer MR, Stintzing FC, Carle R. Cactus Pear Fruits (Opuntia spp.): A Review of Processing Technologies and Current Uses. 2006, J. PACD, 1-25).

DE 103 50 194 offenbart als extrahiertes Pflanzenteil, das weder Cladode noch Frucht ist, die Blüten der Pflanze. Der ethanolische Blütenextrakt soll neuroprotektiv wirken.

Aufgabe und Ziel der vorliegenden Erfindung ist es, Feigenkaktus-Extraktzubereitungen reproduzierbar herzustellen, die verbesserte Eigenschaften aufweisen, insbesondere eine verstärkte hypoglykämische Wirkung zeigen.

Des Weiteren sollen preiswerte, ausreichend verfügbare und neue Pflanzenteile und somit bisher nicht genutzte Rohmaterialien eingesetzt werden.

Überraschendenweise wurde gefunden, dass die Aufgabe gelöst werden kann durch ein Verfahren zur Herstellung von Feigenkaktus-Extraktzubereitungen umfassend die Schritte des
- Extrahieren von Pflanzenteilen, ausgewählt aus Früchten, Fruchtteilen, Cladoden, Cladodenteilen und Mischungen davon mit einem Extraktionsmittel enthaltend Wasser und 0 bis 70 Gew-% Alkohole
- zumindest teilweises Entfernen des Extraktionsmittels.

In einer Ausführungsform umfasst das Verfahren
1. Extraktion von Rohmaterialen a) frische oder getrocknete Fruchtschalenhaut (Trester oder Früchterückstand nach Saftpressung) oder / und frische oder getrocknete Cladoden oder deren Pressrückstände, b) Mischung des Rohmaterials mit alkoholisch-wässrigen Extraktionsmitteln, um einen Rohextrakt zu erhalten, wobei das alkoholisch-wässrigen Extraktionsmittel zwischen 0 und 70 Vol.-% Alkohol enthält;
2. zumindest teilweises Entfernen des alkoholischen Anteils aus dem Rohextrakt, um einen konzentrierten, hauptanteilig wässrigen oder nahezu wasserfreien Dickextrakt zu erhalten;

Die erhaltenen Extraktzubereitung kann unter anderem:
a) unmittelbar zur Herstellung von flüssigen Formulierungen (Sirups, Tropfen, Getränken);
b) zur Füllung von Weichgelatinekapseln oder
c) mit oder ohne Trägerhilfsstoffen zu Trockenextrakten verarbeitet werden kann.

Ein Gegenstand der Erfindung ist die Überführung der Extrakte in Feigenkaktus-Trockenextraktzubereitungen mit pharmazeutisch üblichen Hilfsstoffen in unterschiedlichste Darreichungsformen.

Als Ausgangsmaterial eignen sich insbesondere die Pflanzenteile von Pflanzen, die ausgewählt sind aus der Familie der Cactaceae, speziell aus der Unterfamilie der Opuntioideae, speziell aus dem Tribus der Opuntieae und speziell einer der als Feigenkaktus bezeichneten Arten *Opuntia acanthocarp* Engelmann & Bigelov, *Opuntia aciculata* Griffiths, *Opuntia albicans* Salm-Dyck, *Opuntia anacantha* Speg., *Opuntia arcei* Cárdenas, *Opuntia aurantiaca* Lindl., *Opuntia azurea* Rose, *Opuntia bahiensis* Br. & Rose, *Opuntia basilaris:* Engelmann & Bigelov, *Opuntia berteri* (Colla) Ritter, *Opuntia bigelowii* Engelmann, *Opuntia bispinosa* Backeberg, *Opuntia boliviana, Opuntia bonplandii* (Kunth), *Opuntia cardiosperma* K.Schum., *Opuntia chaffeyi* Br. & Rose, *Opuntia chlorotica* Engelm. & J.M.Bigelow, *Opuntia cochenillifera* (L.) Mill., *Opuntia crassa* Haw., *Opuntia curassavica* (L.) Mill., *Opuntia decumbens* Salm-Dyck, *Opuntia dejecta* Salm-Dyck, *Opuntia delaetiana* (F. A. C. Weber) Vaupel, *Opuntia echios* J.T.Howell, *Opuntia elata* Link & Otto ex Salm-Dyck, *Opuntia elatior* Mill., *Opuntia ellisiana* Griffiths, *Opuntia engelmannii* Salm-Dyck ex Engelm., *Opuntia ficus-indica* (L.) Mill., *Opuntia fragilis* (Nutt.) Haw., *Opuntia gosseliniana* F.A.C.Weber, *Opuntia hyptiacantha* F.A.C.Weber, *Opuntia karwinskiana* Salm-Dyck, *Opuntia lanceolata* (Haw.) Haw., *Opuntia larreyi* F.A.C.Weber ex J.M.Coult. *Opuntia leucotricha* DC., *Opuntia macrocentra* Engelm., *Opuntia macrorhiza* Engelm., *Opuntia megasperma* J.T.Howell, *Opuntia microdasys* (Lehm.) Pfeiff., *Opuntia mieckleyi* K.Schum., *Opuntia monacantha* Haw., *Opuntia phaeacantha* Engelm., *Opuntia pilifera* F.A.C.Weber, *Opuntia polyacantha* Haw., *Opuntia pottsii* Salm-Dyck, *Opuntia puberula* Pfeiff., *Opuntia quitensis* F.A.C.Weber, *Opuntia rastrera* F.A.C.Weber, *Opuntia repens* Bello, *Opuntia robusta* H.L.Wendl. ex Pfeiff., *Opuntia salmiana* J.Parm. ex Pfeiff., *Opuntia santa-rita, Opuntia scheeri* F.A.C.Weber, *Opuntia schickendantzii* F.A.C.Weber, *Opuntia schumannii* F.A.C.Weber ex A.Berger, *Opuntia spinulifera* Salm-Dyck, *Opuntia stenopetala* Engelm., *Opuntia streptacantha* Lem., *Opuntia stricta* (Haw.) Haw., *Opuntia sulphurea* Gillies ex Salm-Dyck, *Opuntia tomentosa* Salm-Dyck , *Opuntia triacanthos* (Willd.) Sweet, *Opuntia tuna* (L.) Mill. (Syn.: *Cactus tuna L.), Opuntia undulata* Griffiths, *Opuntia xvaseyi* (J.M.Coult.) Britton & Rose, *Opuntia velutina* F.A.C.Weber, sowie deren Hybriden.

### Beschreibung des Herstellprozesses

Bei dem erfindungsgemäßen Verfahren werden zunächst die einzeln Feigenkakteen-Rohmaterialen oder die Gemische daraus mit alkoholisch-wässrigen Extraktionsmittel oder mit reinem Wasser extrahiert. Die Pflanzendroge wird hierzu typischerweise zerkleinert (8-12mm), um die Extraktion schnell und erschöpfend zu gestalten. Ein geeignetes Gewichtsverhältnis zwischen Droge und Extraktionsmittel liegt im Bereich von etwa 1:5 bis 1:30, bevorzugt 1:10 bis 1:20.

Dem Fachmann ist bekannt, dass die Extraktion durch ein Erwärmen des Extraktionsmittels verbessert werden kann. Geeignete Temperaturen für die Extraktion liegen zwischen Raumtemperatur und etwa 100°C, bevorzugt im Bereich von 50 bis 90°C.

Typische Extraktionszeiten liegen zwischen 1 und 12 Stunden, bevorzugt 2 bis 6 Stunden.

Im Gegensatz zu den im Stand der Technik beschriebenen Verfahren, wo hoch-gehaltige Alkohol-Wasser-Gemische oder sogar nicht-alkoholische, organische Lösemittel verwendet werden, wird die Extraktion mit einem Extraktionsmittel durchgeführt, das bevorzugt einen geringen Anteil oder keinen Alkohol enthält. Der Gehalt an Alkohol liegt zwischen 0 und 70 Vol.-% des Extraktionsmittels, bevorzugt zwischen 0 und 20 Vol.-%, noch mehr bevorzugt zwischen 0 und 10 Vol.-% Geeignete Alkohole sind insbesondere Ethanol, aber auch Methanol, Isopropanol, mehrwertige Alkohole und Mischungen hiervon.

Die anti-diabetischen Eigenschaften eines Opuntia ficus indica ssp. Blattextraktes aus Wasser sowie aus EtOH 70% V/V sind im Bsp. 1a und 1b beschrieben. Beide Extrakte weisen einen blutzuckersenkenden Effekt auf. Zusätzlich wird die Ausschüttung von körpereigenem Insulin deutlich stimuliert. Der wässrige Extrakt ist hierbei dem wässrig-ethanolischen Auszug (EtOH 70% V/V) überlegen.

Nach der Extraktion wird das Extraktionsmittel vom Drogenrückstand getrennt. Geeignete Verfahren hierfür sind Dekantation, Filtration, Zentrifugalseparation, etc.

Anschließend wird z.B. der alkoholische Anteil des so erhaltenen Rohextraktes ganz oder teilweise entfernt. Dies kann beispielsweise in einem Blasenverdampfer oder einem Plattenverdampfer erfolgen. Durch Zugabe von weiterem Wasser kann der Alkoholanteil reduziert werden, beispielsweise < 2 Gew.-%. Der resultierende Trockensubstanzanteil liegt bei 40 bis 70% (m/m). Die so erhaltenen wässrigen Extrakte werden im Folgenden als Dickextrakt bezeichnet.

Aus den erfindungsgemäß gewonnenen Extrakten oder Dickextrakten können in jedem Konzentrationsgrad weitere Verfahrensschritte zur Abtrennung und Aufreinigung von unwirksamen bzw. wirksamen Bestandteilen angewandt werden.

So bieten sich als Varianten dieser Abtrennung und Aufreinigung zur Steigerung der Wirkung infolge der Freisetzung der wirksamen Prinzipien und Inhaltsstoffe folgende Verfahrensprozesse an:

Hochmolekulare, wenig bioaktive und schlechtlösliche Substanzen, die zunächst in der Primärextraktion mitextrahiert wurden, können mittels Enzymbehandlung (Pektinasen etc.), Membranfiltration sowie Autoklavierung abgebaut oder abgetrennt werden, so dass die löslichen bioaktiveren Substanzen angereichert werden und somit die verbleibenden "nachbehandelten, aufgereinigten Extrakte" eine höhere Wirksamkeit besitzen.

Nach erfolgtem Abtrennungs- und Aufreinigungsverfahren wird Feigenkaktus-Extrakt erhalten, der vom Fachmann in bekannter Weise weiterbehandelt werden kann. Bevorzugt wird der Extrakt getrocknet. Dies kann beispielsweise erfolgen durch Lyophilisierung (Gefriertrocknung), Sprühtrocknung, Vakuumtrocknung, etc. Bevorzugt werden für die Trocknung Hilfsstoffe zugesetzt, um einen rieselfähigen Trockenextrakt zu erhalten. Als Trocknungshilfsstoffe können Maltodextrine, Mono-, Di- und Oligosaccharide (Zucker), Proteingemische und deren Hydrolysate, inkl. Kollagenhydrolysate, Cellulosen und Cellulosederivate, Stärken, Stärkederivate und modifizierte Stärken sowie die insbesondere für Diabetiker geeigneten Hilfsstoffe wie mikrokristalline Cellulosen (MCC), Polyvinylpyrrolidon (PVP), Gummi arabicum oder Fructose eingesetzt werden. Schonende Trocknungsverfahren wie Vakuumtrocknung, Gefriertrocknung oder Sprühtrocknung mit Produkttemperaturen unter 55°C sind bevorzugt.

Gegenstand der Erfindung sind auch pharmazeutisch-technologische bzw. galenische Feigenkaktus-Extraktzubereitungen, die nach dem erfindungsgemäßen Verfahren erhältlich sind, und zur direkten Herstellung der unterschiedlichsten pharmazeutischen Darreichungsformen verwendbar sind.

Überraschenderweise zeigen die erfindungsgemäßen Feigenkaktus-Extraktzubereitungen einen relativ geringen Gehalt an Sterolen (< 100 ppm) sowie insbesondere einen relativ kleinen Gehalt an beta-Sitosterol (< 10 ppm), jeweils bezogen auf den Trockenextrakt, im Vergleich zu Gehalten von 1000 - 10.000 ppm an Sterolen in den getrockneten Drogenteilen. Überraschenderweise wurde somit entdeckt, daß das als blutzuckersenkend beschriebene beta-Sitosterol nicht als Wirkprinzip oder Leitsubstanz herangezogen werden kann. Vielmehr bestimmt der erfindungsgemäß hergestellte Gesamtextrakt die biologische Aktivität.

Die erfindungsgemäßen Extraktzubereitungen weist bevorzugt ein Drogen - Extrakt - Verhältnis (DEV) zwischen 1,5:1 und 20:1, bevorzugt zwischen 2:1 und 5:1 auf.

Gegenstand der Erfindung ist auch ein Arzneimittel, das die erfindungsgemäßen Feigenkaktus-Extraktzubereitungen enthält, sowie die Verwendung der erfindungsgemäßen Feigenkaktus-Extraktzubereitungen zur Herstellung von Arzneimitteln, ergänzenden bilanzierten Diäten, Nahrungsergänzungsmitteln und Medizinprodukten (quellende Ballaststoffe mit Opuntia-Extrakten etc.) zur Prävention und Behandlung von Diabetes Mellitus Typ II, Metabolischem Syndrom und hiermit korrelierten Erkrankungen (diabetische Gefäßerkrankungen, Neuropathie u.a), sowie zur Normalisierung des Blutglukosespiegel und günstiger Beeinflussung des Blutlipidprofils.

Entscheidend ist, dass die hypoglykämischen Eigenschaften beim Diabetiker nicht mit einer "Unterzuckerung" einhergehen, sondern nur zur Normalisierung des Blutzuckerspiegels führen.

Überraschenderweise wurde, neben der neu gefundenen hypoglykämischen Wirkung des erfindungsgemäßen Fruchtschalenhaut-Extraktes, eine deutliche Wirkungssteigerung entdeckt, wenn dieser mit einem Cladoden-Extrakt kombiniert wird. Durch die Kombination der beiden Extrakte wird ein synergistischer Effekt der auch einzeln wirksamen Einzelextrakte belegt (Beispiel 3d)). Dabei ist es entweder möglich, zwei verschiedene Pflanzenteile gemeinsam zu extrahieren oder zwei getrennte Extraktzubereitungen herzustellen, die dann anschließend vermischt werden.

Der erhaltene Extrakt kann in einfacher Weise in eine pharmazeutische Zubereitung überführt werden, beispielsweise in Form von Tabletten, Kapseln, Pastillen, Weichgelatinekapseln oder in flüssige Formulierungen wie Tropfen, Sirupe, Tinkturen oder Urtinkturen. In Nahrungsmitteln sind weitere Darreichungsformen möglich, z.B. in Getränken, Joghurt, Riegeln, Kaugummis, Kaupastillen, Gelatinegummis etc.

Als geeignet haben sich insbesondere Dosierungen von etwa 50 bis 1.000 mg pro Dosiseinheit erwiesen, bevorzugt 100-300 mg, wobei pro Tag bevorzugt etwa 1 bis 3 Dosiseinheiten einzunehmen sind.
Figur 1 zeigt den oralen Glucosetoleranztest in-vivo nach Verabreichung
Figur 2 zeigt die Insulinausschüttung
Figur 3 zeigt den oralen Glucosetoleranztest in-vivo erfindungsgemäß hergestellter Extrakte aus den Cladoden, aus den Früchten sowie einer 50:50 (m/m) Mischung hiervon im Vergleich zur Kontrolle bzw. Glibenclamid.

Die Erfindung wird durch die nachfolgenden Beispiele näher erläutert.

### Beispiel 1a

### Herstellung eines Dickextraktes aus Feigenkaktus-Fruchtschalenhaut:

14,5 kg getrocknete, geschnittene Feigenkaktus-Fruchtschalenhaut wurden mit Wasser im Verhältnis 1:14 versetzt und bei 80°C im Holstein-Kappert-Extrakteur erschöpfend perkoliert. Das Eluat wurde von der Droge abgelassen und über einen 250 µm-Siebbeutel von Drogenresten befreit. Das Perkolat wurde in einem Plattenverdampfer eingedampft. Als Ausbeute resultierten 14,5 kg Dickextrakt mit einem Trockensubstanzanteil von 58,3%.

### Beispiel 1b

### Herstellung eines Dickextraktes aus Feigenkaktus-Cladoden:

12 kg von trockenen, auf 1 cm geschnittenen Feigenkaktus-Cladoden wurden mit Wasser im Verhältnis 1:20 versetzt und bei 80°C im Holstein-Kappert-Extrakteur erschöpfend perkoliert. Das Eluat wurde von der Droge abgelassen und über einen 250 µm Siebbeutel von Drogenresten befreit. Das Perkolat wurde in einem Plattenverdampfer eingedampft. Als Ausbeute resultierten 10,4 kg Dickextrakt mit einem Trockensubstanzanteil von 39,3%.

### Beispiel 1c

### Herstellung eines Dickextraktes aus Feigenkaktus-Cladoden:

10 kg trockene, auf 1 cm geschnittene Feigenkaktus-Cladoden wurden mit Ethanol 70% V/V im Verhältnis 1:14 versetzt und bei 40°C im Holstein-Kappert-Extrakteur erschöpfend perkoliert. Das Eluat wurde von der Droge abgelassen und über einen 250 µm Siebbeutel von Drogenresten befreit. Das Perkolat wurde in einem Plattenverdampfer eingedampft. Als Ausbeute resultierten 3,9 kg Dickextrakt mit einem Trockensubstanzanteil von 50,0%.

### Beispiel 1d

Die Dickextrakte aus Beispiel 1a) und 1b) werden im Nativanteil-Verhältnis 50:50 mittels einer Rührapparatur homogen gemischt.

### Beispiel 2a)

Die Mischung aus Beispiel 1d) wird 15 Minuten bei 121°C autoklaviert.

### Beispiel 2b)

Die Mischung aus Beispiel 1d) wird einer Enzymbehandung mit einer Hemicellulase unterzogen.

### Beispiel 2c)

Die Mischung aus Beispiel 1d) wird über eine Membranfiltration im Gehalt hochmolekularer Substanzen reduziert.

Alle unter Beispiel 2 genannten Aufreinigungsverfahren führen zu einem gleichbleibenden oder verstärkten Auftreten des hypoglykämischen Effektes.

### Beispiel 3a)

### Herstellung eines Trockenextraktes aus Feigenkaktus-Fruchtschalenhaut

Dickextrakt nach Beispiel 1a) wird berechnet auf den Nativanteil mit 30% mikrokristalliner Cellulose versetzt und im Vakuum bei 50°C schonend getrocknet. Nach Mahlung über ein 0,5 mm Sieb entsteht ein homogenes, rieselfähiges Pulver.

### Beispiel 3b)

### Herstellung eines Trockenextraktes aus Feigenkaktus-Cladoden

Dickextrakt nach Beispiel 1b) wird berechnet auf den Nativanteil mit 30% mikrokristalliner Cellulose versetzt und im Vakuum bei 50°C schonend getrocknet. Nach Mahlung über ein 0,5 mm Sieb entsteht ein homogenes, rieselfähiges Pulver.

### Beispiel 3c)

### Herstellung eines Trockenextraktes aus Feigenkaktus-Cladoden

Dickextrakt nach Beispiel 1c) wird berechnet auf den Nativanteil mit 30% mikrokristalliner Cellulose versetzt und im Vakuum bei 50°C schonend getrocknet. Nach Mahlung über ein 0,5 mm Sieb entsteht ein homogenes, rieselfähiges Pulver.

### Bespiel 3d)

### Herstellung eines Trockenextraktes aus Feigenkaktus-Cladoden und Feigenkaktus-Fruchtschalenhaut (50:50 m/m)

Dickextrakt nach Beispiel 1d) (Auszugsmittel Wasser) wird berechnet auf den Nativanteil mit 30% mikrokristalliner Cellulose versetzt und im Vakuum bei 50°C schonend getrocknet. Nach Mahlung über ein 0,5 mm Sieb entsteht ein homogenes, rieselfähiges Pulver.

### Beispiel 3e)

### Herstellung eines Trockenextraktes aus Feigenkaktus-Cladoden und Feigenkaktus-Fruchtschalenhaut (50:50 m/m)

Dickextrakt nach Beispiel 1d) (Auszugsmittel Wasser) wird berechnet auf den Nativanteil mit 30% Fructose versetzt und per Sprühtrocknung schonend getrocknet.

### Beispiel 3f)

### Herstellung eines Trockenextraktes aus Feigenkaktus-Cladoden und Feigenkaktus-Fruchtschalenhaut (50:50 m/m)

Dickextrakt nach Beispiel 1d) (Auszugsmittel Wasser) wird berechnet auf den Nativanteil mit 30% Fructose versetzt und lyophilisiert.

Alle unter Beispiel 3 genannten Trocknungswege eignen sich für eine Überführung in einen Trockenextrakt ohne zu einer signifikanten Reduzierung des hypoglykämischen Effektes zu führen.

### Beispiel 4a)

### Herstellung eines wasserlöslichen Extraktes für die Verwendung in Getränken

Die unter Bespiel 1d) beschriebene wässrige hergestellte Dickextrakt wurde nach Zugabe des Trockenhilfsstoff Maltodextrin im Verhältnis 70% nativer Extrakt : 30% Maltodextrin (m/m) per Sprühtrocknung in eine Trockenextraktzubereitung überführt. Diese Extraktzubereitung ist klar wasserlöslich und kann in Anteil von 1 - 10% Getränkekonzentraten, Limonaden, diätetischen Lebensmittel und Molkereiprodukten zugesetzt werden. Verwendet man anstelle von Maltodextrin als Trockenhilfsstoffe Fructose, oder andere, idealerweise zuckerfreie, wasserlösliche Trägerstoffe mit niedrigem glykämischen Index, wie z.B. Proteinhydrolysaten (Gelitasol o.ä.), so können diese Zubereitungen in Nahrungsprodukten, ergänzende bilanzierte Diäten und in Getränken für Diabetiker verwendet werden

### Beispiele 4b)

### Pektinlutschpastillen

40 Gramm handelsübliches Zitruspektin und 2 Gramm Trinatriumcitrat werden mit 100 Gramm Zuckeraustauschstoff Isomalt gut durchmischt. Diese Mischung wird in 200 ml Wasser eingerührt und unter Rühren aufgekocht, bis sich das gesamte Pektin aufgelöst hat. Jetzt werden weitere 475 g Isomalt und 260 g Fruktose-Sirup zugegeben und auf ca. 80% Trockensubstanzgehalt ausgekocht. Nun werden 50 g einer Feigenkaktus-Trockenextrakt (Beispiel 3e) sowie die Farb- und Aromenstoffe hinzugefügt. Zur Einstellung des pH-Wertes auf 3,4-3,5 werden ca. 17 ml 50%ige Zitronensäurelösung zudosiert. Diese Masse wird bei ca. 95°C in geeignete Formen gegossen, so dass 2 g-Pastillen erhalten werden. Man kann somit ca. 500 Pastillen von je zwei Gramm herstellen. Eine Pektinlutschpastille enthält ca. 100 mg des Feigenkaktus-Extraktes.

### Beispiel 4c)

### Lutschpastillen auf Basis von Gummi Arabicum

In einer Rührapparatur wurden bei 65°C unter Rühren 15 kg Lösung hergestellt, die 33% Gummi Arabicum, 5% Sorbitol, 20% Wasser und 42% Maltitol-Lösung enthält.

Dazu werden 500 Gramm Feigenkaktus-Trockenextrakt (Beispiel 3f)) sowie nach Geschmack abgestimmte Mengen an Aroma und ggf. Süßstoffen eingerührt. Nach vollständigen Homogenisierung wird die Masse in Stärkeformen ausgegossen. Nach dem Trocknen bei 50°C wird das Endgewicht der Pastillen auf 2,0 g eingestellt, die ca. 100 mg Trocknenextraktmischung und 3 mg Lavendelöl enthalten. Die Pastillen werden vom Stärkepulver getrennt und mit Trennmittel behandelt und abgefüllt. Es werden ca. 5000 Pastillen erhalten. Die 2 g-Portion enthält ca. 100 mg des Feigenkaktus-Extraktes.

### Beispiel 4d)

### Kaugummis

100 g Chicle werden gepulvert, mit 300 g Zuckeraustauschstoff Isomalt vermischt und in einer Abdampfschale erhitzt, bis die Masse weich wird. Sie wird dann gut unter Zugabe von 5 g Feigenkaktus-Trockenextrakt (Beispiel 3e)) durchgearbeitet und auf eine mit Stärke bestreute Platte gebracht und bis zur Gleichmäßigkeit geknetet. Eine zusätzliche Aromatisierung kann im vorangegangenen Schritt ebenso erfolgen. Schließlich wird sie in dünne Blätter ausgerollt und noch warm in flache Stangen geschnitten, indem man durch etwas Stärkepulver das Ankleben der Masse an der Platte verhütet. Die Kaugummi-Portionen sollen 2 Gramm betragen und die Portion enthält ca. 100 mg des Feigenkaktus-Extraktes.

### Beispiel 4e)

### Herstellung einer pharmazeutischen Tabletten-Zusammensetzung

Der erfindungsgemäße Extrakt nach Beispiel 3d) wird mit nachfolgender Rezeptur einer Direkttablettierung unterworfen.
300 mg erfindungsgemäßer Feigenkaktus-Trockenextrakt
160 mg mikrokristalline Cellulose
25 mg Natriumcarboxymethylcellulose
10 mg hochdisperses Siliziumdioxid
5 mg Magnesiumstearat

### Beispiel 4f)

### Herstellung einer pharmazeutischen Hartgelatinekapsel-Zusammensetzung

Der erfindungsgemäße Extrakt nach Beispiel 3d) wird mit nachfolgender Rezeptur einer Kapselabfüllung unterworfen.
200 mg Extrakt / Kapsel
50 mg mikrokristalline Cellulose
2 mg hochdisperses Siliciumdioxid

### Beispiel 5

### Studie zur Hypoglykämischen Wirkung im Tiermodell - oraler Glucose Toleranztest (oGTT)

Ein üblicher Weg der Prüfung von Extrakten oder neuen chemischen Verbindungen auf eine Wirkung auf Blutzuckerwerte ist der oGTT (Verspohl, 2002). Hierbei werden unterschiedlichen Gruppen einer Rattenpopulation der Extrakt, eine Blindkontrolle oder ein bekanntes Antidiabetikum, sowie jeweils einer Untergruppe eine intraperitoneale (i.p.) Dosis von Glukose gegeben. Die Extrakte werden 30 Minuten vor der Glukose gegeben. Blutproben werden sublingual zu den Zeitpunkten 0, 15, 30, 60, und 120 Minuten nach der Glukosegabe bzw. 0, 30, 60, 120, und 180 Minuten für die basalen Blutzuckerwerte genommen. Dieses Probenahmesystem stellt sowohl die Überwachung der Auswirkungen der Extrakte auf die basalen Blutzuckerwerte über einen längeren Zeitraum sicher, als auch die Überwachung der Auswirkungen auf die Blutzuckerspiegel nach Glukosebelastung.

Alle getesteten Extrakte, die Blindkontrolle, sowie die Referenz Glibenclamid als orales Antidiabetikum enthielten Propylenglykol als Lösungsvermitteler und wurden oral per Schlundsonde verabreicht

### Versuchstiere

Männliche nicht-hungernde "Wistar"-Ratten mit einem Körpergewicht von 250 - 300g bezogen von Harlan (Indianapolis, IN, U.S.A).

Die nicht-hungernde Verfassung wurde zwecks natürlicherer Physiologie ausgewählt, jedoch variierte hierdurch auch der natürliche Blutglucosewert stärker. Die Ratten wurden zu zweit in Käfigen bei 20 ± 1°C in einem 12-stündigen hell-dunkel-Zyklus gehalten. Wasser und Futter-Pellets waren nach Belieben verfügbar. Gruppen von je 6 Ratten wurden zufällig auf die 12 unterschiedlichen Messgruppen zugeordnet. Alle Versuche wurden in einem ruhigen Raum zwischen 9:00 und 14:00 Uhr durchgeführt. Die Durchführung der Experimente, sowie die Haltung aller Tiere wurde nach den Grundsätzen und Leitlinien des Institutional Animal Care und Use Comittee (IACUC) (University of Florida, Gainesville, USA) durchgeführt

### Material

Glibenclamid (=Glyburide, Sigma-Aldrich, St. Louis, U.S.A) wurde als bekanntes Referenz-Antidiabetikum in einer Dosierung von 18mg/kg entsprechend der Literatur eingesetzt (Subash Babu et al., 2007; Verspohl et al., 2005). Es wurde bei einem Propylenglykol-Gehalt von 0,5% mit deionisiertem Wasser auf 18mg/5ml verdünnt.

Die Messextrakte wurden in 5 ml deionisiertem Wasser mit 0,5% Propylenglykol suspendiert. Alle Messlösungen wurden täglich frisch zubereitet. Alle Tiere wurden mindestens 30 Minuten vor der Testung in den Testraum verbracht und verblieben dort während des ganzen Tests. Die Glucose (Sigma-Aldrich) wurde (mit Hilfe von Ultraschall) in einer 0,9%-igen Salzlösung in einer Konzentration von 2g/5ml gelöst und 30 Minuten nach der Applikation der Testlösung i.p. verabreicht

### Blutglucosebestimmung

Blut wurde zu den entsprechenden Zeitpunkten aus der sublingualen Vene nach einer Halothan-Anästhesie entnommen und bei 4°C mit Heparin-Zusatz aufbewahrt.

Die Proben wurden bei 8600 U/min für 10 Minuten zentrifugiert. Das überstehende Plasma wurde anschließend mittels eines autoanalyzer (Merck, Darmstadt) untersucht. Analytische Plasmakontrollen und Blindwerte wurden verwendet, um genaue Ergebnisse innerhalb der spezifizierten Grenzen zu garantieren.

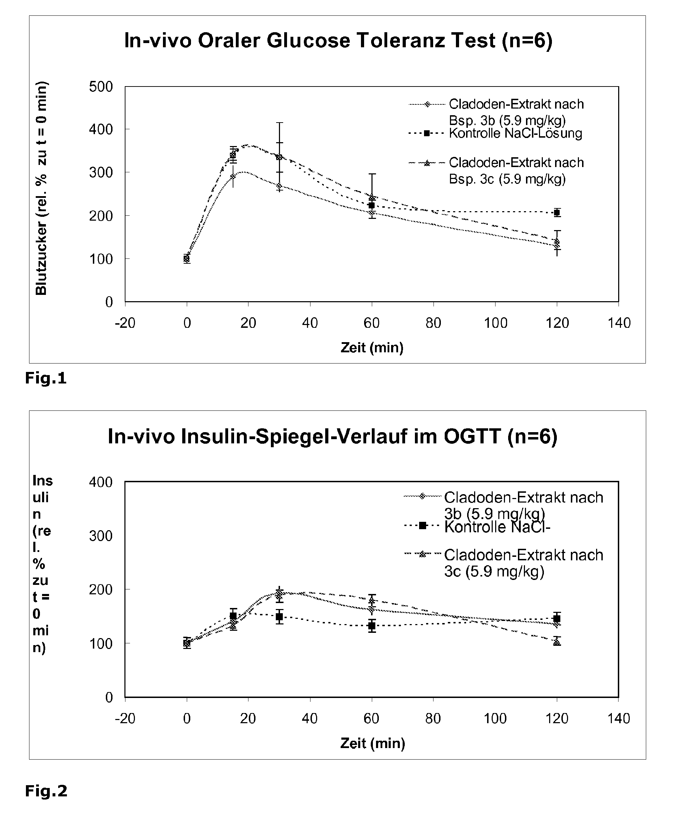

Figur 1 zeigt den hypoglykämischer Effekt nach Gabe von von zwei Trockenextrakten aus Feigenkaktus-Cladoden im Vergleich zu NaCl-Lösung (Kontroll-wert) im OGTT-Modell an Ratten (n=6). Eine Reduzierung des Blutzuckers gegenüber unbehandelten Tieren war messbar. Extrakt 3b) war mit einer Senkung bis zu 20% deutlich potenter als Extrakt 3c (-3%).

Figur 2 zeigt den Verlauf der Plasma-Insulinspiegel nach Gabe von zwei Trockenextrakten aus Feigenkaktus-Cladoden im Vergleich zu NaCl-Lösung (Kontrollwert) im OGTT-Modell an Ratten (n=6). Eine gesteigerte InsulinAusschüttung (+ 20%) gegenüber unbehandelten Tieren war messbar.

### Statistik und Berechnungen

Gehalte und Kinetik-Daten wurden analysiert mit einseitiger Varianzanalyse (ANOVA) und Student-Newman-Keuls multiplem Vergleichstest unter Verwendung der Software Graphpad 4.0 (San Diego, USA). Die Fläche unter der Kurve (AUC) wurde nach der Trapez-Methode ohne Erweiterung über den letzten Messpunkt bestimmt.

### Ergebnis

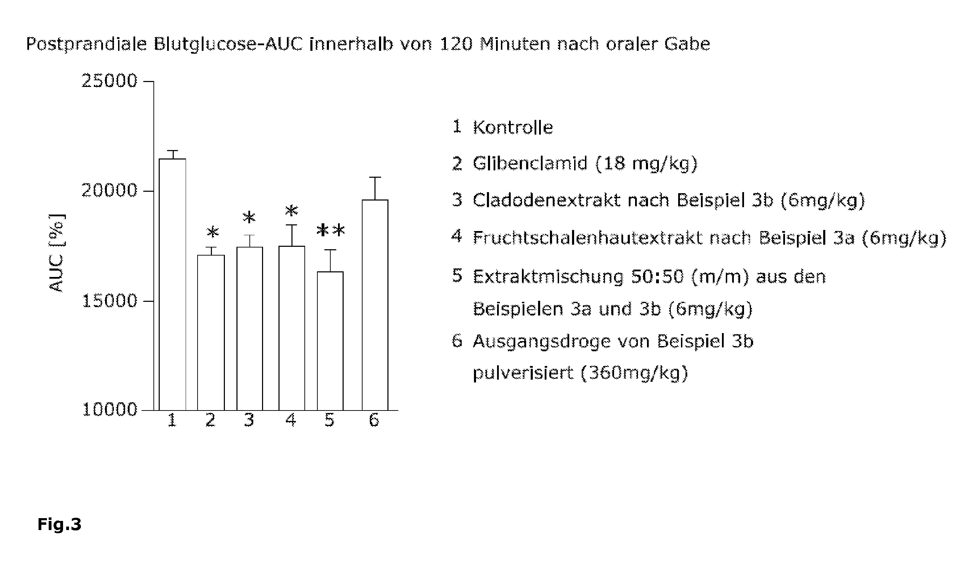

Das Testmodell bestätigt für die Referenzsubstanz Glibenclamid die Eignung zur Messung eines blutzuckersenkenden Effektes gegenüber der Kontrollgruppe. Beide Einzelextrakte aus den Feigenkaktus-Cladoden und den Feigenkaktus-Fruchtschalen zeigen ebenfalls deutlich hypoglykämische Effekte. Demgegenüber weißt die pulversierte Ausgangsdroge (Cladoden) auch in deutlich höherer Dosis keinen signifikanten Effekt auf. Eine Mischung aus verschiedenen Drogenteilen des Feigenkaktusses, hier getestet eine 50:50-Mischung aus Cladoden und Fruchtschalen, zeigt überraschenderweise eine synergistische Wirkung gegenüber den Einzelextrakten (siehe Figur 3). Dies weißt auf verschiedene Wirkmechanismen hin.

Figur 3 zeigt den Effekt von Glibenclamid und der erfindungsgemäßen Extrakte im OGTT (Oral Glucose Tolerance Test) ausgedrückt in Prozent AUC, bei n = 5-6 pro Messgruppe; Signifikanzniveau vs. Kontrolle: * p< 0,05, ** p< 0,01.

## Patentansprüche

1. Verfahren zur Herstellung einer Extraktzubereitung von Feigenkakteen, umfassend die Schritte:
- Extrahieren von Pflanzenteilen, ausgewählt aus Früchten, Fruchtteilen, Cladoden, Cladodenteilen und Mischungen davon mit einem Extraktionsmittel enthaltend Wasser und 0 bis 70 Gew-% Alkohole
- zumindest teilweises Entfernen des Extraktionsmittels.

2. Verfahren nach Anspruch 1, **dadurch gekennzeichnet, dass** die Feigenkakteen Opuntia ficus indica ssp. sind.

3. Verfahren nach Anspruch 1 oder 2, **dadurch gekennzeichnet, dass** die Pflanzenteile frisch oder getrocknet sind.

4. Verfahren nach mindestens einem der Ansprüche 1 bis 3, **dadurch gekennzeichnet, dass** die Pflanzenteile ausgewählt werden aus Fruchtschalenhaut, Trester, Fruchtrückstand nach Saftpressung, Pressrückstände von Cladoden und Mischungen davon:

5. Verfahren nach mindestens einem der Ansprüche 1 bis 4, **dadurch gekennzeichnet, dass** mindestens ein Pflanzteil aus Früchten und mindestens ein Pflanzenteil aus Cladoden gemeinsam extrahiert werden.

6. Verfahren nach mindestens einem der Ansprüche 1 bis 5, **dadurch gekennzeichnet, dass** als Extraktionsmittel Wasser mit einem Alkohol ausgewählt aus Methanol, Ethanol, n-Propanol, Isopropanol, Glykol, Glycerin und Mischungen hiervon verwendet wird.

7. Verfahren nach mindestens einem der Ansprüche 1 bis 6, **dadurch gekennzeichnet, dass** das Extraktionsmittel 0 bis 20, bevorzugt 0 bis 10 Gew-% Alkohole enthält.

8. Verfahren nach mindestens einem der Ansprüche 1 bis 7, **dadurch gekennzeichnet, dass** der Extrakt zu einem Dickextrakt eingeengt oder zum Trockenextrakt, gegebenenfalls unter Zusatz von Trockenhilfsstoffen, getrocknet wird.

9. Verfahren nach mindestens einem der Ansprüche 1 bis 8, **dadurch gekennzeichnet, dass** nach dem Extrahieren eine enzymatische Behandlung, insbesondere mit Pektinasen, Cellulasen und/oder Hemicellulasen durchgeführt wird.

10. Verfahren nach mindestens einem der Ansprüche 1 bis 9, **dadurch gekennzeichnet, dass** nach der Extraktion eine Membranfiltration durchgeführt wird.

11. Verfahren nach mindestens einem der Ansprüche 8 bis 10, **dadurch gekennzeichnet, dass** die Trocknung unter schonenden Bedingungen durch temperatur- und druckregulierte Verfahren wie Gefriertrocknung, Sprühtrocknung oder Vakuumtrocknung durchgeführt wird.

12. Extraktzubereitung erhältlich durch ein Verfahren nach einem der Ansprüche 1 bis 11.

13. Extraktzubereitung nach Anspruch 12, **dadurch gekennzeichnet, dass** mindestens zwei Extrakte vermischt werden, wobei mindestens ein Extrakt aus Früchten oder Fruchtteilen und mindestens ein Extrakt aus Cladoden oder Cladodenteilen gewonnen wird.

14. Lebensmittel, diätetisches Lebensmittel, Nahrungsergänzungsmittel oder Arzneimittel enthaltend eine Extraktzubereitung nach einem der Ansprüche 12 oder 13.

15. Verwendung einer Extraktzubereitung nach mindestens einem der Ansprüche 12 oder 13 zur Herstellung eines Lebensmittels, eines diätetischen Lebensmittels, eines Nahrungsergänzungs- oder Arzneimittels zur Prophylaxe oder Behandlung von Diabetes mellitus II, zur Steigerung der körpereigenen Insulinausschüttung, zur günstigen Beeinflussung von Parametern des metabolischen Syndroms, zur günstigen Beeinflussung des Blutglucosespiegels, zur günstigen Beeinflussung des Blutlipidprofils, sowie zur Behandlung von Störungen des Magensäurehaushaltes und säureabhängiger Ulcera in Ventriculus und Duodenum.
